# EUROPEAN PATENT APPLICATION

(11) **EP 4 438 788 A1**
(43) Date of publication of application: **02.10.2024**
(21) Application number: 22897979.5
(22) Date of filing: 29.11.2022
(51) Int. Cl.: D06F 35/00, D06F 39/00, A61L 2/10

(54) **CLOTHING TREATMENT APPARATUS**

(30) Priority: 29.11.2021 CN 202111432536; 29.11.2021 CN 202111432809; 29.11.2021 CN 202111435804; 29.11.2021 CN 202111436675; 29.11.2021 CN 202111432360
(71) Applicant: QINGDAO HAIER LAUNDRY ELECTRIC APPLIANCES CO., LTD., Qingdao, Shandong 266101 (CN); Haier Smart Home Co., Ltd., Qingdao, Shandong 266101 (CN)
(72) Inventor: ZHAO, Zhiqiang, Qingdao, Shandong 266101 (CN); XU, Sheng, Qingdao, Shandong 266101 (CN)
(74) Representative: Winter, Brandl - Partnerschaft mbB
(86) International application number: PCT/CN2022/134916
(87) International publication number: WO 2023/093898

(57) **Abstract**

A laundry treatment apparatus in the present application includes a laundry treatment barrel and a light plasma generation unit having an air chamber. The air chamber is internally provided with a photoplasma tube for irradiating air in the air chamber. An air inlet of the air chamber is in communication with the external air; and an air outlet of the air chamber is in communication with an interior of the laundry treatment barrel via an air outlet pipeline. In the present application, the external air in the air chamber irradiated by the photoplasma tube is generated a sterilizing gas which is blown into the laundry treatment barrel via the air outlet pipeline, thereby sterilizing and deodorizing the interior of the laundry treatment barrel and laundry inside to completely kill microorganisms and efficiently remove unpleasant odors, especially a sterilizing effect is better for laundry being no-resistant to high temperature.

## Description

### Field

The application relates to a field of laundry treatment apparatus, and in particular relates to a laundry treatment apparatus with a sterilizing function.

### Background

Laundry is often in contact with the outside, so pathogenic bacteria adhere to the laundry. Common pathogenic bacteria include Escherichia coli and Staphylococcus aureus and so on, which may cause to decrease in the body's resistance and increase in the risk of illness. Therefore, on the basis of cleaning of the surface of laundry, more and more users also require that laundry can be sterilized.

Many existing laundry treatment apparatuses are provided with sterilization device which usually sterilize laundry by one or more ways such as ultraviolet rays, silver ions, high temperature and ozone. Among them, ultraviolet rays can penetrate the air to sterilize laundry; however, ultraviolet rays easily cause damage to human eyes and skin. Silver ions can inhibit the growth of mold and deodorize. But if absorbed by the internal organs of the human body, silver ions are accumulated to cause pathological changes. High temperature treatment can inactivate pathogenic bacteria; but laundry made of silk, wool and other materials that are not resistant to high temperatures are easily deformed. Excessive ozone can strongly stimulate the respiratory tract of people, to cause symptoms such as sore throat, chest tightness and coughing, etc. In addition, a pressurized ionization makes part of oxygen gas in air change into ozone, and nitrogen gas be ionized to produce nitrogen oxides which can cause cancer to human.

Thus, the present application is proposed.

### Summary

The technical problem to be solved by the present application is how to overcome the deficiencies of the prior art. A laundry treatment apparatus is provided to improve the sterilization efficiency of a laundry treatment apparatus. Another aspect of the present application is to provide a laundry treatment apparatus to sterilize laundry containing materials that are not resistant to high temperatures.

In order to solve the above problems, the present application adopts the following basic idea of technical solution.

A laundry treatment apparatus, includes a laundry treatment barrel and an optical plasma generation unit with an air chamber. An air inlet of the air chamber is communicated with an external air, and a photoplasma tube is arranged in the air chamber for irradiating air being inhaled in the air chamber. An air outlet of the air chamber is communicated with an interior of the laundry treatment barrel via an air outlet pipeline.

As an embodiment, the laundry treatment apparatus further includes a housing. The laundry treatment barrel is disposed inside the housing, and an optical plasma generation unit is arranged between the housing and the laundry treatment barrel.

As an embodiment, an air inlet end of the air outlet pipeline is connected with the air chamber, and an air outlet end of the air outlet pipeline is connected with an upper front side of the laundry treatment barrel, so that the external air in the air chamber irradiated by the photoplasma tube is blown into the laundry treatment barrel through the air outlet pipeline.

As an embodiment, a first air inlet is provided on the upper front side of the laundry treatment barrel. The air outlet pipeline is communicated with the interior of the laundry treatment barrel through the first air inlet.

Optionally, the air outlet pipeline is provided with an air outlet valve for controlling to open and close the air outlet pipeline.

As an embodiment, a door seal is arranged between the housing and a front flange of the laundry treatment barrel. An air outlet end of the air outlet pipeline is connected with the door seal, such that the external air in the air chamber irradiated by the photoplasma tube is blown into the laundry treatment barrel through the air outlet pipeline.

As an embodiment, a front of the laundry treatment barrel has an opening, and an upper of the door seal corresponding to an upper front side of the laundry treatment barrel is provided with a second air inlet. The air outlet pipeline is communicated with the opening on the front of the laundry treatment barrel through the second air inlet.

As an embodiment, a fan is arranged in the air chamber for taking external air into the air chamber through the air inlet of the air chamber, and discharging the external air in the air chamber irradiated by the photoplasma tube into the air outlet pipeline through an air outlet of the air chamber.

As an embodiment, the fan is arranged on a position close to the air inlet of the air chamber, and the photoplasma tube is arranged on a position close to the air outlet of the air chamber.

The application provides a laundry treatment apparatus, including a housing, a laundry treatment barrel disposed in the housing, and an optical plasma generation unit with an air chamber. An air inlet and an air outlet of the air chamber are communicated with an interior of the laundry treatment barrel through an air inlet pipeline and an air outlet pipeline respectively to form a circulating air flow. A photoplasma tube for irradiating the circulating air flow is disposed in the air chamber.

As an embodiment, an air inlet end of the air inlet pipeline is connected with a upper rear side of the laundry treatment barrel, and the air inlet end of the air inlet pipeline is connected with the air chamber, so that air inside the laundry treatment barrel is discharged into the air chamber through the air inlet pipeline.

An air inlet end of the air outlet pipeline is connected with the air chamber. A door seal is provided between the housing and a front flange of the laundry treatment barrel, and an air outlet end of the air outlet pipeline is connected with an upper front side of the laundry treatment barrel or the door seal, so that a circulating air in the air chamber irradiated by thephotoplasma tube is blown into the laundry treatment barrel through the air outlet pipeline.

As an embodiment, a radial dimension of the air outlet pipeline is greater than a radial dimension of the air inlet pipeline.

As an embodiment, an air outlet is provided on an upper rear side of the laundry treatment barrel, and the air inlet pipeline is communicated with the interior of the laundry treatment barrel through the air outlet.

Optionally, an air inlet valve for controlling to open and close the air inlet pipeline is arranged in the air inlet pipeline.

As an embodiment, a first air inlet is provided on an upper front side of a laundry treatment apparatus, and the air outlet pipeline is communicated with an interior of the laundry treatment apparatus through the first air inlet.

Optionally, an air outlet valve for controlling to open and close of the air outlet pipeline is arranged in the air outlet pipeline.

As an embodiment, an opening is formed on a front of the laundry treatment apparatus. An upper of a door seal corresponding to an upper front of the laundry treatment apparatus is provided with a second air inlet. The air outlet pipeline is communicated with the opening on the front of the laundry treatment apparatus through the second air inlet.

Preferably, an air outlet valve for controlling to open and close of the air outlet pipeline is arranged in the air outlet pipeline.

As an embodiment, a fan is arranged in the air chamber for taking external air into the air chamber through the air inlet of the air chamber, and discharging the external air in the air chamber irradiated by the photoplasma tube into the air outlet pipeline through an air outlet of the air chamber.

As an embodiment, the air chamber is connected with an air inlet valve for controlling to open and close the air inlet of the air chamber, and/or an air outlet valve for controlling to open and close the air outlet of the air chamber.

As an embodiment, the air chamber includes a first chamber and a second chamber which are arranged in front and back direction and communicated with each other. A fan is arranged in the first chamber, and a photoplasma tube is arranged in the second chamber. The first chamber has the air inlet of the air chamber, and the second chamber has the air outlet of the air chamber.

As an embodiment, the fan is arranged on a position close to the air inlet of the first chamber, and the photoplasma tube is arranged on a position close to the air outlet of the second chamber.

As an embodiment, the air outlet of the air chamber is formed on a bottom of the second chamber. An upper end of the air inlet pipeline is connected with the bottom of the air chamber.

The application provides a laundry treatment apparatus, including a laundry treatment barrel and an optical plasma generation unit with an air chamber. An air outlet of the air chamber is communicated with the laundry treatment barrel through the air outlet pipeline. An air inlet of the air chamber is communicated with the laundry treatment barrel through a first air inlet branch or communicated with an external air through a second air inlet branch.

As an embodiment, the air chamber has a first air inlet and a second air inlet. The first air inlet of the air chamber is communicated with the laundry treatment barrel through the first air inlet branch, and the second air inlet of the air chamber is communicated with the external air through the second air inlet branch.

As an embodiment, the first air inlet branch is provided with a first air inlet valve for controlling to open and close the first air inlet branch, and/or the second air inlet branch is provided with a second air inlet valve for controlling to open and close the second air inlet branch.

As an embodiment, the first air inlet branch has a first air inlet and an air outlet. The first air inlet branch between the first air inlet and the air outlet is provided with a second air inlet for communicating with the external air. The first air inlet branch between the second air inlet and the air outlet is configured to form the second air inlet branch.

As an embodiment, the laundry treatment apparatus includes an external connection pipeline connected with the second air inlet branch. The second air inlet is communicated with the external air through the external connection pipeline.

As an embodiment, a second air inlet valve is disposed in the second air inlet or in the external connection pipeline to control the second air inlet branch to be connected with or disconnected from the external air.

The first air inlet branch between the first air inlet and the second air inlet is provided with a first air inlet valve for controlling to open and close the first air inlet branch.

As an embodiment, a side of the first air inlet branch away from the second air inlet branch or the second air inlet is provided with a three-way valve. The first air inlet branch between the first air inlet and the second air inlet is communicated with the external air and the second air inlet branch through the three-way valve.

As an embodiment, one end of the first air inlet branch is connected with an upper rear side of the laundry treatment barrel, and the other end is connected with the air chamber.

One end of the air outlet pipeline is connected with the air chamber, and the other end is connected with an upper front side of the laundry treatment barrel or connected with the door seal on the upper front side of the laundry treatment barrel.

As an embodiment, a photoplasma tube and a fan are arranged in the air chamber. The photoplasma tube is used to irradiate air in the air chamber. The fan is used to take air in the first air inlet branch or the second air inlet branch into the air chamber.

The application provides a laundry treatment apparatus, including a laundry treatment barrel and a drying air duct for allowing hot air to flow into the laundry treatment barrel. Both ends of the drying air duct are respectively connected with the front and the rear of the laundry treatment barrel, and the drying air duct is provided with a photoplasma tube for irradiating the hot air in the drying air duct.

As an embodiment, the drying air duct is provided with an installation hole. The photoplasma tube is arranged in the drying air duct by passing a lower end the photoplasma tube through the installation hole.

As an embodiment, a fan is arranged in the drying air duct. The drying air duct has an air duct inlet and an air duct outlet. The fan is arranged on a position of the drying air duct close to the air duct inlet, and the photoplasma tube is arranged on a position of the drying air duct close to the air duct outlet.

As an embodiment, a heating pipe is arranged in the drying air duct, and between the fan and the photoplasma tube, so that the hot air in the drying air duct flows toward the photoplasma tube.

As an embodiment, the drying air duct is provided with an air inlet valve for controlling to open and close the air duct inlet. The air inlet valve is arranged between the fan and the air duct inlet, so that air in the laundry treatment barrel is configured to flow into the drying air duct through the air duct inlet.

As an embodiment, an air inlet end of the drying air duct is connected with an upper rear side of the laundry treatment barrel, so that air in the laundry treatment barrel is configured to flow backward and into the drying air duct through the air duct inlet.

As an embodiment, the drying air duct is provided with an air outlet valve for controlling to open and close the air duct outlet. The air outlet valve is arranged between the photoplasma tube and the air duct outlet, so that the hot air in the drying air duct irradiated by the photoplasma tube is discharged into the laundry treatment barrel through the air duct outlet.

As an embodiment, a laundry treatment apparatus includes a housing, and a laundry treatment barrel arranged in the housing. A door seal is arranged between the housing and a front flange of the laundry treatment barrel. The air outlet end of the drying air duct is connected with an upper front side of the laundry treatment barrel or the door seal, so that the hot air in the drying air duct irradiated by the photoplasma tube is blown into the laundry treatment barrel through the air duct outlet.

As an embodiment, a laundry treatment apparatus includes a control device. The control device is connected with the photoplasma tube to control the photoplasma tube to irradiate the hot air in the drying air duct.

The present application provides a laundry treatment apparatus, including a housing, an optical plasma generation unit, and a laundry treatment barrel arranged in the housing.

The optical plasma generation unit includes:
a shell, in which an air chamber is formed;
a photoplasma tube for irradiating air in the air chamber;
a connecting pipeline, wherein, an air inlet end of the connecting pipeline is connected with an air outlet of the air chamber, an air outlet end of the connecting pipeline is communicated with an interior of the laundry treatment barrel through an air outlet pipeline; and
a blocking member, being configured to control to be connected with or disconnected from the air chamber, such that the blocking member reciprocates along an inside of the connecting pipeline.

As an embodiment, the connecting pipeline includes an inclined pipeline section extending obliquely upward along an air flow direction. The blocking member is arranged in the inclined pipeline section.

A fan is arranged in the air chamber for guiding air in the air chamber to flow into the connecting pipeline. Air is configured to flow through the inclined pipeline section to drive the blocking member to move upwards, so that the connecting pipeline is communicated with the air chamber.

As an embodiment, an inner peripheral wall of the inclined pipeline section is provided with a limiting rib extending in a radial direction of the inside of the inclined pipeline section. Air is configured to flow through the inclined pipeline section to drive the blocking member to move upward and be in contact with the limiting rib, so that the blocking member is stopped moving upward.

As an embodiment, an inner diameter of the inclined pipeline section is gradually increased in an air flow direction.

As an embodiment, part of the shell is recessed toward an interior of the air chamber to form an accommodating chamber having an opening in a horizontal direction. An elastic member is arranged in the accommodating chamber and in interference fit with the accommodating chamber. An insertion hole is formed in the elastic member.

As an embodiment, a photocatalyst excitation layer is in the shell, and the photoplasma tube is in opposite to the photocatalyst excitation layer. Alternately a photocatalyst excitation layer is provided inside the photoplasma tube.

As an embodiment, an ultraviolet tube has UVC ultraviolet bands and/or UVD ultraviolet bands.

As an embodiment, a beam is arranged on a top of the housing, and fixedly connected with a mounting bracket. The shell of an optical plasma generation unit is detachably connected with the mounting bracket through a plug-in structure.

Optionally, the beam and the mounting bracket are respectively provided with a connecting hole. A fastener is configured to pass through the connecting hole on the beam and the connecting hole on the mounting bracket to connect the beam with the mounting bracket.

As an embodiment, part of the shell is recessed toward the interior of the air chamber to form an accommodating chamber being in interference fit with the elastic member. The elastic member is provided with an insertion hole. The mounting bracket is provided with a stick matching with the insertion hole.

After adopting the above technical solutions, there are the following advantages in the present application compared with the prior art.
1. In the present application, an external air sucked into the air chamber is generated a sterilizing gas by a irradiation of the photoplasma tube, and flows into an interior of a laundry treatment barrel through an air outlet pipeline, thereby flowing inside the laundry treatment barrel. The inside of the laundry treatment barrel and the laundry in the laundry treatment barrel are sterilized, especially a sterilizing effect is better for laundry being no-resistant to high temperature.
2. In the present application, an air outlet valve is provided on an air outlet pipeline, so that the air outlet valve controls to open or close the air outlet pipeline, and the air chamber is controlled to be connected with or disconnected from the laundry treatment barrel. The sterilizing gas is more accurately blown into laundry treatment barrel 2 according to the use of the laundry treatment apparatus or the actual needs of users.
3. In the present application, the air chamber is connected with an air inlet valve and/or an air outlet valve for controlling to open or close the air inlet and/or air outlet of the air chamber, thereby controlling the air chamber to be connected with or disconnected from the external air and/or the air outlet pipeline. So the optical plasma generation unit can better generate a sterilizing gas, and the sterilizing efficiency of the laundry treatment apparatus is improved.
4. In the present application, the photoplasma tube has UVC ultraviolet and/or UVD ultraviolet. UVD ultraviolet bands can efficiently excite oxygen and water in air to generate light plasma clusters, and UVC ultraviolet bands have an efficient bactericidal effect, so that microorganisms attached to the laundry treatment barrel and laundry therein are completely killed.

The specific implementations of the present application are further described in detail below in conjunction with the accompanying drawings.

### Description of drawings

In accompanying figures:
Fig. 1 is a partial sectional view of a laundry treatment apparatus in an embodiment of the present application;
Fig. 2 is a partial sectional view of a laundry treatment apparatus in another embodiment of the present application;
Fig. 3 is a partial sectional view of a laundry treatment apparatus in another embodiment of the present application;
Fig. 4 is a partial sectional view of a laundry treatment apparatus in another embodiment of the present application;
Fig. 5 is a partial sectional view of a laundry treatment apparatus in another embodiment of the present application;
Fig. 6 is a partial sectional view of a laundry treatment apparatus in another embodiment of the present application;
Fig. 7 is a partial sectional view of a laundry treatment apparatus in another embodiment of the present application;
Fig. 8 is a partial sectional view of a laundry treatment apparatus in another embodiment of the present application;
Fig. 9 is a partial sectional view of a laundry treatment apparatus in another embodiment of the present application;
Fig. 10 is a partial sectional view of a laundry treatment apparatus in another embodiment of the present application;
Fig. 11 is a partial sectional view of a laundry treatment apparatus in another embodiment of the present application;
Fig. 12 is a partial sectional view of a laundry treatment apparatus in another embodiment of the present application;
Fig. 13 is a partial view of a structural diagram of a laundry treatment device in an embodiment of the present application;
Fig. 14 is a structural diagram of a connection of an optical plasma generation unit in an embodiment of the present application;
Fig. 15 is a structural diagram of a connection of an optical plasma generation unit from another perspective in an embodiment of the present application;
Fig. 16 is a structural diagram of a connection of an optical plasma generation unit from another perspective in an embodiment of the present application;
Fig. 17 is a cross-sectional view of the A-A direction in a state where an air chamber and a connecting pipeline are in communication with each other in Fig. 16;
Fig. 18 is a cross-sectional view along A-A in a state where an air chamber is disconnected from a connecting pipeline in Fig. 16.

### Detailed description

### Embodiment 1

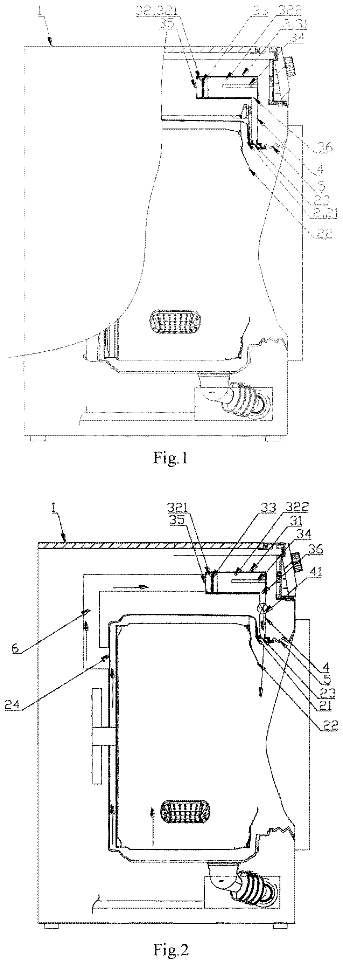

As shown in Figs. 1 to Fig. 11, an embodiment of the present application provides a laundry treatment apparatus, including a laundry treatment barrel 2 and an optical plasma generation unit 3 with an air chamber 32. An air inlet 35 of the air chamber 32 is communicated with atmosphere of the outside, and inside the air chamber 32 is provided with a photoplasma tube 34 for irradiating the atmosphere of the outside being inhaled. An air outlet 35 of the air chamber 32 is communicated with an inside of the laundry treatment barrel 2 via an air outlet pipeline 4.

In the embodiment, in a case of power-on, the optical plasma tube 34 can emit optical plasma and ion clusters. The optical plasma tube 34 is configured to irradiate the external atmosphere inhaled into the air chamber 32.The optical plasma and ion clusters make oxygen gas and water molecules in air be decomposed into hydroxyl ions, free oxygen atoms, superoxide ions and other oxidants to form a sterilizing gas, which gas can not only sterilize the laundry treatment barrel 2 and laundry inside thereof, but also decompose harmful impurities in air inside the laundry treatment barrel 2 to become inert compounds, such as carbon dioxide and water. The peculiar smell of the clothes is quickly and easily removed.

As shown in Fig. 1, in the embodiment, the laundry treatment apparatus further includes a housing 1, the laundry treatment barrel 2 is disposed inside the housing 1, and an optical plasma generation unit 3 is arranged between the housing 1 and the laundry treatment barrel2.

In the embodiment, the air in the housing 1 is configured to flow into the air chamber 32 through the air inlet 35 of the air chamber 32, and the photoplasma tube 34 is configured to irradiate the air flowing into the air chamber 32 to form hydroxyl ions, free oxygen atoms, superoxide ions and other oxidants being as a sterilizing gas.

As shown in Figs. 1 and 2, in the embodiment, an air inlet end of the air outlet pipeline 4 is connected with the air chamber 32, and an air outlet end of the air outlet pipeline 4 is connected with an upper front side of the laundry treatment barrel2, so that the external air irradiated by the photoplasma tube 34 in the air cha2mber 32 is blown into the laundry treatment barrel 2 through the air outlet pipeline 4.

In the embodiment, the air inlet end of the air outlet pipeline 4 is connected with the optical plasma generation unit 3, and the air outlet end is connected with the upper front side of the laundry treatment barrel 2.The sterilizing gas from the optical plasma generation unit 3 is flown into the air outlet pipeline 4, and then flows in the inside of the laundry treatment barrel 2 from the front, inhibiting the reproduction of microorganisms attached to the laundry treatment barrel 2 and laundry. Meanwhile, the harmful impurities in the air inside the laundry treatment barrel 2 and the odorous substances attached to laundry are decomposed into an inert compound.

As shown in Figs. 1 and 2, in the embodiment, a first air inlet 23 is provided on the upper front side of the laundry treatment barrel 2, and the air outlet pipeline 4 is communicated with the interior of the laundry treatment barrel 2 through the first air inlet 23.

In the embodiment, the air outlet pipeline 4 is directly communicated with the laundry treatment barrel 2 through the first air inlet 23 on the laundry treatment barrel 2, so that the sterilizing gas in the air outlet pipeline 4 can be efficiently blown into the interior of the laundry treatment barrel 2.

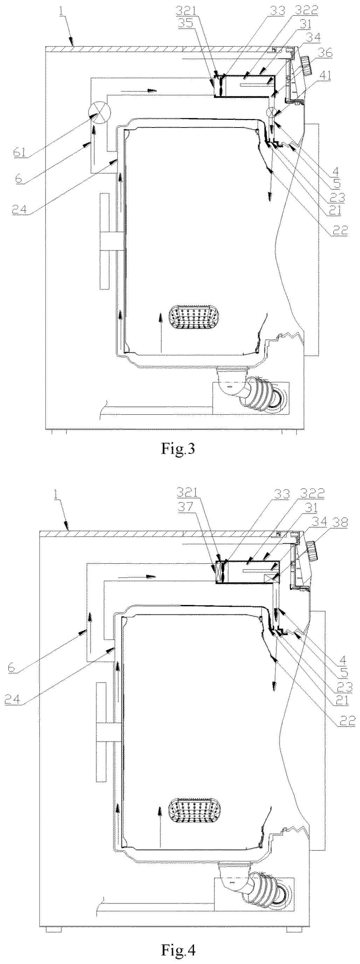

As shown in Fig. 3, in the embodiment, the air outlet pipeline 4 is provided with an air outlet valve 41 for controlling to open and close the air outlet pipeline 4, so that the air chamber 32 is controlled to be connected with or disconnected from the laundry treatment barrel 2.The sterilizing gas is more accurately controlled to flow into the laundry treatment barrel 2 through the air outlet pipeline 4 according to the use of the laundry treatment barrel or the actual needs of user.

In the embodiment, the laundry treatment apparatus can be a washing machine, a laundry dryer or other apparatus with a laundry treatment function. A laundry treatment barrel 2 can be a single barrel, and can also include an outer barrel 21 and an inner barrel 22 installed inside and being coaxial with the outer barrel 21.

In the embodiment, as an example that the laundry treatment barrel 2 is a single barrel, laundry to be treated is placed inside the laundry treatment barrel 2, and the sterilizing gas in the air outlet pipeline 4 flows directly into the laundry treatment barrel 2 through the first air inlet 23 and flow from the front of the laundry treatment barrel 2 to the interior of the laundry treatment barrel2.

In the embodiment, as an example that the laundry treatment barrel 2 includes an outer barrel 21 and an inner barrel 22, the air outlet pipeline 4 is connected with the upper front side of the outer barrel 21, and a first air inlet 23 is formed on the upper front side of the outer barrel 21. The sterilizing gas in the air outlet pipeline 4 flows into the interior of the outer barrel 21 through the first air inlet 23.A front portion of the outer barrel 21 and a front portion of the inner barrel 22 all have openings, so the sterilizing gas flows from the outer barrel 21 to the inner barrel 22.

### Embodiment 2

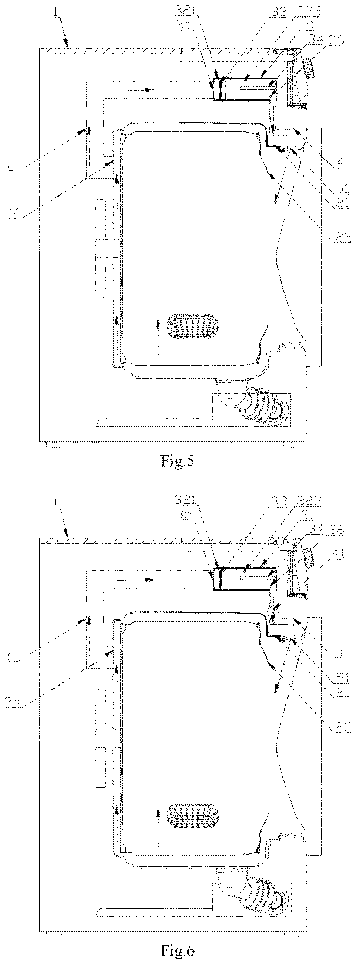

As shown in Figs. 5 to 8, the difference between the embodiment and embodiment 1 is that a door seal 5 is arranged between the housing 1 and a front flange of the laundry treatment barrel 2, and an air outlet end of an air outlet pipeline 4 is connected with the door seal 5.So the external air in the air chamber 32 irradiated by the photoplasma tube 34 is blown into the laundry treatment barrel 2 through the air outlet pipeline 4, and the sterilizing gas flows in an interior of the laundry treatment barrel 2.

As shown in Figs. 5 to 8, the front of the laundry treatment barrel 2 has an opening, and an upper of the door seal 5 corresponding to the upper front side of the laundry treatment barrel 2is provided with a second air inlet 51. The air outlet pipeline 4 is communicated with the opening on the front of the laundry treatment barrel 2 through the second air inlet 51.

In the embodiment, the air outlet pipeline 4 is communicated with the laundry treatment barrel 2 through the second air inlet 51 of the door seal 5, and the sterilizing gas in the air outlet pipeline 4 flows into the interior of the laundry treatment barrel 2 through the opening on the front of the laundry treatment barrel2.

As shown in Fig. 5, in the embodiment, the air outlet pipeline 4 is provided with an air outlet valve 41 for controlling to open and close the air outlet pipeline 4, so that the air chamber 32 is controlled to be connected with or disconnected from the laundry treatment barrel 2.The sterilizing gas is more accurately controlled to flow into the laundry treatment barrel 2 according to the use of the laundry treatment barrel or the actual needs of user.

In the embodiment, the laundry treatment apparatus can be a washing machine, a laundry dryer or other apparatus with a laundry treatment function. A laundry treatment barrel 2 can be a single barrel, and can also include an outer barrel 21 and an inner barrel 22 installed inside and being coaxial with the outer barrel 2.

In the embodiment, as an example that the laundry treatment barrel 2 is a single barrel, laundry to be treated is placed inside the laundry treatment barrel 2, and the sterilizing gas in the air outlet pipeline 4 flows directly into the laundry treatment barrel 2 through the second air inlet 51 of the door seal 5 and flow from the front of the laundry treatment barrel 2 to the interior of the laundry treatment barrel2.

In the embodiment, as an example that the laundry treatment barrel 2 includes an outer barrel 21 and an inner barrel 22, the air outlet pipeline 4 is connected with the door seal 5, and a position of the door seal 5 corresponding to the upper front side of the laundry treatment barrel 2is provided with the second air inlet 51. The sterilizing gas in the air outlet pipeline 4 flows into the interior of the outer barrel 21 through the second air inlet 51 of the door seal 5.A front portion of the outer barrel 21 and a front portion of the inner barrel 22 all have openings, so the sterilizing gas flows from the outer barrel 21 to the inner barrel 22.

### Embodiment 3

As shown in Figs. 1 to 11, the embodiment further describes the solutions based on Embodiments 1 and 2.A fan 33 is arranged in the air chamber 32 for taking external air into the air chamber 32 through the air inlet 35 of the air chamber 32, and discharging the external air irradiated by the photoplasma tube 34 in the air chamber 32 into the air outlet pipeline 4 through an air outlet 36 of the air chamber 32.

As shown in Figs. 1 to 11, in the embodiment, the optical plasma generation unit 3 includes a shell 31 which is configured to surround an air chamber 32.The shell 31 is provided with an air inlet 35 of the air chamber 32 and an air outlet 36 of the air chamber 32.

As shown in Figs. 3 and 6, the air chamber 32 is connected with an air inlet valve 37 for controlling to open and close the air inlet 35 of the air chamber 32, and/or connected with an air outlet valve 38 for controlling to open and close the air outlet 36 of the air chamber 32, so that the air chamber 32 can be opened and closed.

In the embodiment, the air chamber 32 is connected with an air inlet valve 37 and/or an air outlet valve 38 to control to open or close the air inlet 35 and/or the air outlet 36 of the air chamber 32, thereby controlling the air chamber 32 to be connected with or disconnected from the outside and/or the air outlet pipeline 4. So the optical plasma generating unit 3 can better generate the sterilizing gas, to improve the sterilizing efficiency of a laundry treatment apparatus.

As shown in Figs. 1 to 11, in the embodiment, the fan 33 is arranged on a position close to the air inlet 35 of the air chamber 32, and the photoplasma tube 34 is arranged on a position close to the air outlet 36 of the air chamber 32.

As shown in Figs. 1 to 11, in the embodiment, the air chamber 32 includes a first chamber 321 and a second chamber 322 which are communicated with each other. The fan 33 is arranged in the first chamber 321, and the photoplasma tube 34 is arranged in the second chamber 322.The air inlet 35 is formed on a wall of the first chamber 321, and the air outlet 36 is formed on a wall of the second chamber 322, so that the first chamber 321 is communicated with the second chamber 322.

In the embodiment, an optical plasma concentration detection device is provided in the air chamber 32 or in the laundry treatment barrel 2.The optical plasma concentration detection device is used to detect whether the light plasma concentration in the air chamber 32, or the laundry treatment barrel2 containing the sterilizing gas meets a sterilization requirement, or not. The light plasma concentration detection device is a concentration sensor.

### Embodiment 4

As shown in Figs. 1 to 11 , an embodiment of the present application provides a laundry treatment device, including a housing 1, a laundry treatment barrel 2 disposed in the housing 1, and an optical plasma generation unit 3 with an air chamber 32. An air inlet 35 and an air outlet 36 of the air chamber 32 are communicated with the interior of the laundry treatment barrel 2 through an air inlet pipeline 6 and an air outlet pipeline 4 respectively to form a circulating air flow. A photoplasma tube 34 for irradiating the circulating air flow is disposed in the air chamber 32.

As shown in Fig. 2 to Fig. 8, in the embodiment, the air inlet end of the air inlet pipeline 6 is connected with an upper rear side of the laundry treatment barrel 2, and the air inlet end of the air inlet pipeline 6 is connected with the air chamber 32, so gas inside the laundry treatment barrel 2 is discharged into the air chamber 32 through the air inlet pipeline 6.

In the embodiment, the sterilizing gas is configured to sterilize the inside of the laundry treatment barrel 2 and laundry disposed in the laundry treatment barrel, and flows from the front to the rear of the laundry treatment barrel 2. During the flowing process, the effect of the sterilizing gas is gradually weakened, and the gas is discharged into the air inlet pipeline 6 and flows into the air chamber 32 through the air inlet pipeline 6.

As shown in Figs. 2 to 8, in the embodiment, an air outlet 24 is provided on a upper rear side of the laundry treatment barrel 2, and the air inlet pipeline 6 is communicated with the interior of the laundry treatment barrel 2 through the air outlet 24.

In the embodiment, the air inlet pipeline 6 is directly connected with the laundry treatment barrel2 through the air outlet 24 on the laundry treatment barrel2.Gas in the laundry treatment barrel 2 is discharged into the air inlet pipeline 6from the air outlet 24 on the upper rear side of the laundry treatment barrel 2, and then discharged into the air chamber 32 of the optical plasma generation unit 3.

As shown in Fig. 3 and Fig. 6, in the embodiment, the air inlet pipeline 6 is provided with an air inlet valve 61 for controlling to open and close the air inlet pipeline 6, and the air chamber 32 is controlled to be connected with or disconnected from the air inlet pipeline 6. Air in the laundry treatment barrel 2 is blown into the air chamber 32 to ensure to sterilizing the inside of the laundry treatment barrel 2, according to the use of the laundry treatment barrel or the actual needs of user.

As shown in Figs. 2 to 8, in the embodiment, the air inlet end of the air outlet pipeline 4 is connected with the air chamber 32.A door seal 5 is provided between the housing 1 and a front flange of the laundry treatment barrel 2, and the air outlet end of the air outlet pipeline 4 is connected with the upper front side of the laundry treatment barrel2 or the door seal 5, so that the circulating air in the air chamber 32 irradiated by the photoplasma tube 34 is blown into the laundry treatment barrel2 through the air outlet pipeline 4.

As shown in Figs. 2 to 5, in the embodiment, the air inlet end of the air outlet pipeline 4 is connected with the optical plasma generation unit 3, and the air outlet end of the air outlet pipeline 4 is connected with the upper front side of the laundry treatment barrel 2. The sterilizing gas from by the optical plasma generation unit 3 is blown into the air outlet pipeline 4, and then flows into the laundry treatment barrel 2from the front thereof, so as to sterilize and deodorize the inside of the laundry treatment barrel 2 and laundry.

As shown in Figs. 5 to 8, in the embodiment, the air inlet end of the air outlet pipeline 4 is connected with the optical plasma generation unit 3, and the air outlet end of the air outlet pipeline 4 is connected with the door seal 5.The sterilizing gas from by the optical plasma generation unit 3 is blown into the air outlet pipeline 4, and then flows into the laundry treatment barrel 2from the door seal 5.

As shown in Figs. 2 to 8, in the embodiment, a radial dimensionof the air outlet pipeline 4 is greater than a radial dimension of the air inlet pipeline 6, which is conducive to the rapid flow of circulating air. So air in the air inlet pipeline 6 is quickly blown into the air chamber 32, and the light plasma generation unit 3 is used to sterilize and deodorize the airflow from the interior of the laundry treatment barrel 2.Thereby the airflow is rapidly circulated.

### Embodiment 5

As shown in Figs. 2 to 5, the embodiment is further described based on the above-mentioned Embodiment 4.A first air inlet 23 is provided on an upper front of a laundry treatment barrel 2, and an air outlet pipeline 4 is communicated with an interior of the laundry treatment barrel 2through the first air inlet 23, so that a circulating air irradiated by a photoplasma tube 34 in an air chamber 32 is blown into the interior of the laundry treatment barrel 2 through the air outlet pipeline 4.

As shown in Fig. 3 and Fig. 4, in the embodiment, the air outlet pipeline 4 is provided with an air outlet valve 41 for controlling to open and close the air outlet pipeline 4, so the air chamber 32 is controlled to be connected with or disconnected from the laundry treatment barrel 2. The sterilizing gas can be more accurately blown into the laundry treatment barrel 2 according to the use of the laundry treatment apparatusor the actual needs of user.

In the embodiment, as an example that the laundry treatment barrel 2 is a single barrel, laundry to be treated is disposed inside the laundry treatment barrel 2.The sterilizing gas in the air outlet pipeline 4 is directly blown into the laundry treatment barrel 2through the first air inlet 23 on the laundry treatment barrel 2, and flows into the interior of the laundry treatment barrel 2from the front of the laundry treatment barrel 2, so as to sterilize and deodorize the inside of the laundry treatment barrel 2 and laundry. Air in the laundry treatment barrel 2 is discharged into the air inlet pipeline 6 through an air outlet 24 at the rear of the laundry treatment barrel 2, and flows into the air chamber 32 through the air inlet pipeline 6to form a circulating airflow. The photoplasma tube 34 is configured to irradiate the circulating airflow.

In the embodiment, as an example that the laundry treatment barrel 2 includes an outer barrel 21 and an inner barrel 22, the air outlet pipeline 4 is connected with the upper front side of the outer barrel 21.A first air inlet 23 is formed on the upper front side of the outer barrel 21. The sterilizing gas in the air outlet pipeline 4 is blown into an interior of the outer barrel 21 through the air outlet pipeline 4.The front of the outer barrel 21 and the front of the inner barrel 22 all have openings, and so the sterilizing gas can flows into the inner barrel 22from the outer barrel 21 to sterilize and deodorize the inside of the inner barrel 22 and laundry. The upper rear side of the outer barrel 21 is provided with an air outlet 24, and air inside the inner barrel 22 flows downward into the outer barrel 21 through the opening at the front of the inner barrel 22.Air inside the outer barrel 21 flows to the rear from the front and is discharged into the air inlet pipeline 6 through the air outlet 24 on the upper rear side of the outer barrel 21, and flows into the air chamber 32 from the air inlet pipeline 6 to form the circulation air flow. The photoplasma tube 34 is configured to irradiate the circulating airflow.

### Embodiment 6

As shown in Figs. 4 to 8, the embodiment is further described based on the above-mentioned embodiment 4.An opening is formed on the front of the laundry treatment barrel 2, and an upper of a door seal 5 corresponding to the upper front of the laundry treatment barrel 2 is provided with a second air inlet 51.The air outlet pipeline 4 is communicated with the opening on the front of the laundry treatment barrel 2 through the second air inlet 51, so that the air outlet pipeline 4 is connected withthe laundry treatment barrel 2.

In the embodiment, the air inlet end of the air outlet pipeline 4 is connected to the optical plasma generation unit 3, and the air outlet end of the air outlet pipeline 4 is connected with the door seal 5.The sterilizing gas from the optical plasma generation unit 3 is blown into the air outlet pipeline 4, and then flows into the interior of the laundry treatment barrel 2 through the door seal 5, to inhibit the reproduction of microorganisms attached to the laundry treatment barrel 2 and laundry, and at the same time. Meanwhile, the harmful impurities in air in the laundry treatment barrel 2 and the odorous substances attached to laundry are decomposed into an inert compound.

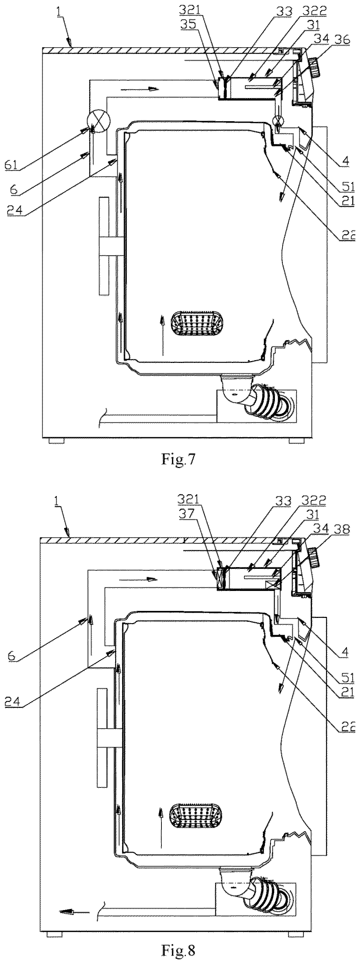

As shown in Figs. 6 and 7, in the embodiment, the air outlet pipeline 4 is provided with an air outlet valve 41 for controlling to open and close the air outlet pipeline 4, so the air chamber 32 is controlled to be connected with or disconnected from the laundry treatment barrel 2 through the door seal 5. The sterilizing gas can be more accurately blown into the laundry treatment barrel 2 according to the use of the laundry treatment apparatus or the actual needs of user.

### Embodiment 7

As shown in Figs. 1 to 8, the embodiment is further described based on the above mentioned Embodiments 4-6.The air chamber 32 is also provided with a fan 33 for taking gas in the air inlet pipeline 6 into the air chamber 32through an air inlet 35 of the air chamber 32, and for discharging the circulating air irradiated in the air chamber 32 by the photoplasma tube 34 into the air outlet pipeline 4 through an air outlet 36 of the air chamber 32.

As shown in Figs. 2 to 6, in the embodiment, the optical plasma generation unit 3 includes a shell 31 which is configured to surround an air chamber 32.The shell 31 is provided with the air inlet 35 of the air chamber 32 and the air outlet 36of the air chamber 32.

As shown in Figs. 4 and 8, in the embodiment, the air chamber 32 is connected with an air inlet valve 37 for controlling to open and close the air inlet 35 of the air chamber 32, and/or an air outlet valve 38for controlling to open and close the air outlet 36 of the air chamber 32, so that the air chamber 32 is connected with or disconnected from the air inlet pipeline 6 and/or the air outlet pipeline 4.Thereby the optical plasma generating unit 3 can better generate the sterilizing gas.

As shown in Figs. 2 to Fig. 8, in the embodiment, the air chamber 32 includes a first chamber 321 and a second chamber 322 which are arranged in front and back direction and communicated with each other. A fan 33 is arranged in the first chamber 321, and a photoplasma tube 34 is arranged in the second chamber 322.The first chamber 321 has the air inlet 35 of the air chamber 32, and the second chamber 322 has the air outlet 36 of the air chamber 32.

As shown in Figs. 1 to 8, in the embodiment, the fan 33 is arranged on a position close to the air inlet 35 of the first chamber 321, and the photoplasma tube 34 is arranged on a position close to the air outlet 36 of the second chamber 322.

As shown in Figs. 1 to 8, in the embodiment, the air outlet 36 of the air chamber 32 is formed ona bottom of the second chamber 322, and the upper end of the air inlet pipeline 6 is connected with the bottom of the air chamber 32.

As shown in Figs. 1 to 8, in the embodiment, the air outlet pipeline 4 is configured to be extended in a vertical direction, so the sterilizing gas rapidly flows into the laundry treatment barrel 2, and the efficiency of sterilizing the laundry treatment apparatus is improved.

### Embodiment 8

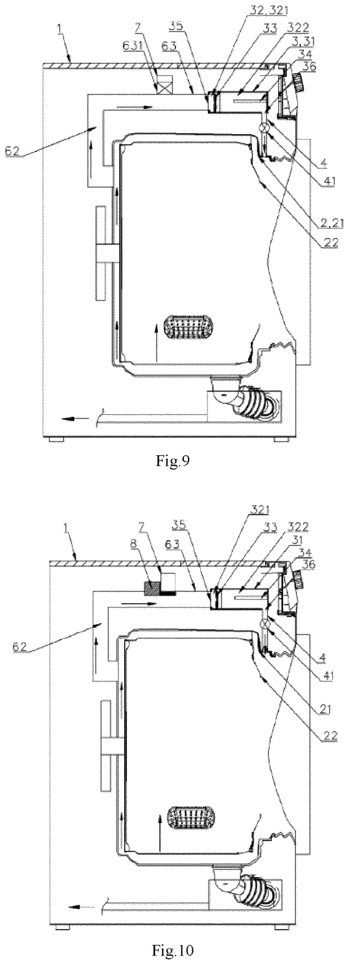

As shown in Figs. 9 to 11, an embodiment of the present application provides a laundry treatment apparatus, including a laundry treatment barrel 2 and an optical plasma generation unit 3 with an air chamber 32. An air outlet 36 of the air chamber 32 is communicated with the laundry treatment barrel 2 through the air outlet pipeline 4.An air inlet 35 of the air chamber 32 is communicated with the laundry treatment barrel 2 through a first air inlet branch 62 or communicated with the outside atmosphere through a second air inlet branch 63.

As shown in Figs. 9 to 11, in the embodiment, the first air inlet branch 62 has a first air inlet 23 and an air outlet 24. The first air inlet branch 62 between the first air inlet 23 and the air outlet 24 is provided with the second air inlet 51 for communicating with the external air. The first air inlet branch 62 between a second air inlet 51 and the air outlet 24 is configured to form the second air inlet branch 63.

In the embodiment, both ends of the first air inlet branch 62 are connected with the laundry treatment barrel 2 and the air chamber 32 respectively. The first air inlet 23 for communicating with the laundry treatment barrel 2 and the air outlet 24 for communicating with the air chamber 32 are respectively formed on both ends of the first air inlet branch 62, so that the first air inlet branch 62, the optical plasma generation unit 3, the air outlet pipeline 4 and the laundry treatment apparatus 2 form an air circulation system. The second air inlet branch 63 is a part of the first air inlet branch 62.The external air flows into the second air inlet branch 63 through the second air inlet 51, and then flows into the air chamber 32 through the air inlet 35 of the air chamber 32, to realize air circulation.

As shown in Figs. 9 to 11, in the embodiment, the laundry treatment apparatus includes an external connection pipeline 7 connected with the second air inlet branch 63.The second air inlet 51 is communicated with the external air through the external connection pipeline 7, so that the external air flows into the second air inlet branch 63 through the external connection pipeline 7, and the external air can quickly flow into the second air inlet branch 63.

As shown in Fig. 9, a second air inlet valve 631 is disposed in the second air inlet 51 or in the external connection pipeline 7 to control the second air inlet branch 63 to be connected with or disconnected from the external air, thereby controlling the air chamber 32 to be connected with or disconnected from the external atmosphere. The laundry treatment barrel 2 and laundry can be sterilized and deodorized according to the use of the laundry treatment apparatus or the actual needs of user.

As an embodiment, the first air inlet branch 62 between the first air inlet 23 and the second air inlet 51 is provided with a first air inlet valve 621 for controlling to open and close the first air inlet branch 62, thereby controlling the air chamber 32 to be connected with or disconnected from the interior of the laundry treatment barrel 2.

As shown in Figs. 9 to 11, in the embodiment, one end of the first air inlet branch 62 is connected with the upper rear side of the laundry treatment barrel 2, and the other end is connected with the air chamber 32.

As shown in Figs. 9 to 11, in the embodiment, one end of the air outlet pipeline 4 is connected with the air chamber 32, and the other end is connected with the upper front side of the laundry treatment barrel 2 or connected with the door seal 5 on the upper front side of the laundry treatment barrel 2.

In the embodiment, the air inlet end of the air outlet pipeline 4 is connected with the optical plasma generating unit 3, and the air outlet end of the air outlet pipeline 4 is connected with the upper front side of the laundry treatment barrel 2. The sterilizing gas is blown into the air inlet pipeline 6, and then flows into the laundry treatment barrel 2 from the front of the laundry treatment apparatus.

In the embodiment, the laundry treatment apparatus further includes a housing 1. The optical plasma generating unit 3 is arranged between the housing 1 and the laundry treatment barrel 2.Air in the housing 1 flows into the second air inlet branch 63 through the second air inlet 51.

In the embodiment, the door seal 5 is provided between the housing 1 and a front flange of the laundry treatment barrel 2.A front of the laundry treatment barrel 2 has an opening. The air inlet end of the air outlet pipeline 4 is connected with the optical plasma generating unit 3,and the air outlet end of the air outlet pipeline 4 is connected with the door seal 5.The sterilizing gas from the optical plasma generating unit 3 is blown into the air outlet pipeline 4, and then flows into the laundry treatment barrel 2 through the opening of the front of the laundry treatment barrel 2 through the door seal 5.

As shown in Figs. 9 to 11, in the embodiment, a diameter of the first air inlet branch 62 is larger than a diameter of the air outlet pipeline 4, so as to make the circulating air rapidly flow. Air in the air outlet pipeline 4 rapidly flows into the air chamber 32, and air inside the laundry treatment barrel 2 is sterilized and deodorized by the optical plasma generating unit 3.

In the embodiment, as an example that the laundry treatment barrel 2 is a single barrel. Laundry to be treated is placed inside the laundry treatment barrel 2.The sterilizing gas from the optical plasma generation unit 3 is blown into the laundry treatment barrel 2 through the air outlet pipeline 4, and flows into the interior of the laundry treatment barrel 2 from the front of the laundry treatment barrel 2 to sterilize and deodorize the inside of the laundry treatmentbarrel2 and laundry. Air in the laundry treatment barrel2 flows to the rear of the laundry treatment barrel2 and is discharged into the first air inlet branch 62, and then flows into the air chamber 32 from the first air inlet branch 62, to form a circulating air flow.

In the embodiment, as an example that the laundry treatment barrel 2 includes an outer barrel 21 and an inner barrel 22.The sterilizing gas from the optical plasma generation unit 3 is blown into the outer barrel 21 through the air outlet pipeline 4.Both the front of the outer barrel 21 and the front of the inner barrel 22 have openings, and the sterilizing gas flows downward into the inner barrel 22 to sterilize and deodorize the inside of the inner barrel 22 and laundry. The air inside the inner barrel 22 flows downward and is discharged into the outer barrel 21 through the opening on the front of the inner barrel 22. Air inside the outer barrel 21 flows to the rear of the laundry treatment barrel 2 and is discharged into the first air inlet branch 62, and flows into the air chamber 32 from the first air inlet branch 62, to form a circulating airflow.

### Embodiment 9

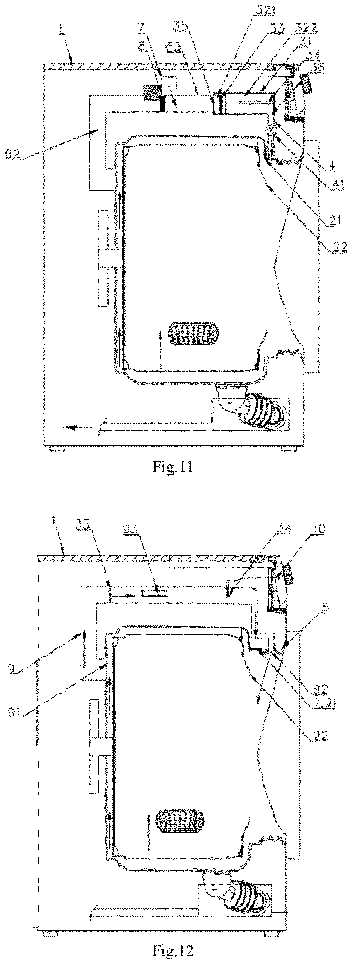

As shown in Fig. 10 and Fig. 11, the difference between the embodiment and the above-mentioned Embodiment 8 is that: a side of the first air inlet branch 62 away from the second air inlet branch 63 or the second air inlet 51 is provided with a three-way valve 8. The first air inlet branch 62 between the first air inlet 23 and the second air inlet 51 is communicated with the external atmosphere and the second air inlet branch 63 through the three-way valve 8.

In the embodiment, the second air inlet 51 is controlled to be closed by the three-way valve 8, so that the first air inlet branch 62 is connected with the air chamber 32.The first air inlet branch 62, the optical plasma generation unit 3, the air outlet pipeline 4 and the laundry treatment barrel 2 form an air circulation system. The first air inlet branch 62 is controlled to be blocked by the three-way valve 8, so that the external atmosphere is communicated with the second air inlet branch 63, and the external atmosphere is communicated with the air chamber 32.Thereby fresh air is blown to the interior of the laundry treatment barrel 2.

### Embodiment 10

The difference between the embodiment and the above Embodiment 8 is that: the air chamber 32 has a first air inlet 35 and a second air inlet 35. The first air inlet 35 of the air chamber 32 is communicated with the laundry treatment barrel 2 through the first air inlet branch 62, and the second air inlet 35 of the air chamber 32 is communicates with the external atmosphere through the second air inlet branch 63.

In the embodiment, the first air inlet branch 62 is communicated with the air chamber 32 through the first air inlet 35, so as to take air inside the laundry treatment barrel 2 into the air chamber 32 of the optical plasma generation unit 3. The second air inlet branch 63is communicated with the air chamber 32 through the second air inlet 35, so as to taking the external air into the air chamber 32 of the optical plasma generation unit 3.It is ensured that the first air inlet branch 62 and the second air inlet branch 63 are independent of each other, and the external air is prevented from flowing back into the laundry treatment barrel 2 through the first air inlet branch 62.

In the embodiment, the first air inlet branch 62 is provided with a first air inlet valve 621 for controlling to open and close the first air inlet branch 62, and/or the second air inlet branch 63 is provided with a second air inlet valve 631 for controlling to open and close the second air inlet branch 63.

In the embodiment, the first air inlet valve 621 is configured to control to open and close the first air inlet branch 62, and control the first air inlet branch 62 to be connected with or disconnected from the air chamber 32.The second air inlet valve 631 is configured to control to open and close the second air inlet branch 63, and then control the air chamber 32 is connected with or disconnected from the external atmosphere. The laundry treatment barrel 2 and laundry therein are sterilized and deodorized according to the use of the laundry treatment apparatus or the actual needs of user.

### Embodiment 11

As shown in Fig. 12, an embodiment of the present application provides a laundry treatment apparatus with a function of drying laundry, including a laundry treatment barrel 2 and a drying air duct 9 for allowing hot air to flow into the laundry treatment barrel 2. Both ends of the drying air duct 9 are respectively connected with the front and rear ends of the laundry treatment barrel 2, and the drying air duct 9 is provided with a photoplasma tube 34 for irradiating the hot air in the drying air duct 9.

In the embodiment, air in the drying air duct 9 and air in the laundry treatment barrel 2 forms a circulating air flow. Air inside the laundry treatment barrel 2 flows into the drying air duct 9 and is heated to form hot air. The hot air in the drying air duct 9 is irradiated by the photoplasma tube 34, so that the hot air with sterilizing and deodorizing functions is generated in the drying air duct 9 and then diffused to the interior of the laundry treatment barrel 2 for sterilizing and deodorizing the laundry.

As shown in Fig. 12, in the embodiment, the drying air duct 9 is provided with an installation hole. A lower end the photoplasma tube 34 passes through the installation hole, and the photoplasma tube 34is arranged in the drying air duct 9.

In the embodiment, the photoplasma tube 34 is arranged in the drying air duct 9 through the installation hole. When the hot air flows through the photoplasma tube 34, the light plasma and ion clusters emitted by the photoplasma tube 34 make oxygen and water molecules in air be decomposed into hydroxide ions, free oxygen atoms, superoxide ions and other oxidants, and a certain amount of ozone is generated. Under the action of hot air, ozone is easily decomposed by heat, thus it is ensured that the environment is disinfected and sterilized by the light plasma with low ozone concentration.

As shown in Fig. 12, in the embodiment, a fan 33 is arranged in the drying air duct 9.The drying air duct 9 has an air duct inlet 91 and an air duct outlet 92.The fan 33 is arranged on a position of the drying air duct close to the air duct inlet 91, and the photoplasma tube 34 is arranged on a position of the drying air duct 9 close to the air duct outlet 92.

In this embodiment, the fan 33 is configured to make air in the drying air duct 9 and air in the laundry treatment barrel 2 flows to form a circulating air flow. When the air flows in the drying air duct 9, the photoplasma tube 34 is configured to irradiate air to form a sterilizing gas containing oxides to sterilize a laundry treatment apparatus.

As shown in Fig. 12, in the embodiment, a heating pipe 93 is also arranged in the drying air duct 9, and between the fan 33 and the photoplasma tube 34.When air in the laundry treatment barrel 2 flows into the air duct 9, air is first heated by heating pipe 93 to form the drying hot air, and then the drying hot air flows to the photoplasma tube 34, so that it is beneficial to reduce the ozone concentration in the sterilizing gas.

In the embodiment, the light plasma and ion clusters emitted by the photoplasma tube 34 make oxygen and water molecules in air are decomposed into hydroxide ions, free oxygen atoms, superoxide ions and other oxidants. However, with the generation of ozone, excessive ozone can strongly stimulate the respiratory tract of people, to cause symptoms such as sore throat. Ozone is decomposed in the hot air in the drying air duct 9, reducing the ozone concentration in the circulating airflow.

As shown in Fig. 12, in the embodiment, the heating pipe 93 is in cylindrical-shape. The heating pipe 93 is horizontally arranged in the drying air duct 9, and is configured to extend along a flow direction of air in the drying air duct 9, to ensure that the heat of air is more fully exchanged with the heating pipe 93.

As shown in Fig. 12, in the embodiment, the drying air duct 9 is provided with an air inlet valve 61 for controlling to open and close the air duct inlet 91.The air inlet valve 61 is arranged between the fan 33 and the air duct inlet 91, so that air in the laundry treatment barrel 2 is configured to flow into the drying air duct 9 through the air duct inlet 91.

As shown in Fig. 12, in the embodiment, the air inlet end of the drying air duct 9 is connected with an upper rear side of the laundry treatment barrel 2, so that air in the laundry treatment barrel 2 is configured to flow backward and into the drying air duct 9through the air duct inlet 91. The hot and humid air flowing into the drying air duct 9is dried by the heating pipe 93.The photoplasma tube 34 sterilizes and deodorizes the dried air.

In the embodiment, the hot air in the drying air duct 9 is configured to sterilize and deodorize the laundry treatment barrel 2 and laundry. In the process of the hot air flowing to the rear of the laundry treatment barrel 2 from the front of the laundry treatment barrel2, moisture in laundry is carried in the drying air to form a high-humidity air, so drying, sterilizing and deodorizing effects are gradually weakened. Finally the high-humidity air is discharged into the drying air duct 9 through the air duct inlet 91 on the upper rear part of the laundry treatment barrel 2.

As shown in Fig. 12, in the embodiment, the drying air duct 9 is provided with an air outlet valve 41 for controlling to open and close the air duct outlet 92.The air outlet valve 41 is arranged between the photoplasma tube 34 and the air duct outlet 92, so that the hot air irradiated in the drying air duct 9 by the photoplasma tube 34 is discharged into the laundry treatment barrel 2 through the air duct outlet 92.

In the embodiment, the air outlet valve 41 is configured to control to open or close the air duct outlet 92. So the drying air duct 9 is controlled to be connected with or disconnected from the laundry treatment barrel 2.The laundry treatment barrel 2 and the laundry is sterilized and deodorized according to the use of the laundry treatment apparatus or the actual needs of users.

As shown in Fig. 12, in the embodiment, a laundry treatment apparatus includes a housing 1, and the laundry treatment barrel 2 is arranged in the housing 1.A door seal 5 is arranged between the housing 1 and a front flange of the laundry treatment barrel2.The air outlet end of the drying air duct 9 is connected with the upper front side of the laundry treatment barrel 2 or the door seal 5, so that the hot air in the drying air duct 9 irradiated by the photoplasma tube 34 is blown into the laundry treatment barrel 2 through the air duct outlet 92.

In the embodiment, the air outlet end of the drying air duct 9 is connected with the upper front side of the laundry treatment barrel 2 or the door seal 5, so that the hot air with the functions of sterilization and deodorization flows into the interior of the laundry treatment barrel 2 through the air duct outlet 92 of the drying air duct 9, to inhibit the growth of microorganisms attached on the laundry treatment barrel 2 and laundry inside the laundry treatment barrel 2, and remove the peculiar smell of laundry.

As shown in Fig. 12, in the embodiment, an inner diameter of part of the drying air duct 9corresponding to the air inlet end is greater than an inner diameter of part of the drying air duct 9corresponding to the air outlet end, which is conducive to the rapid circulation of the air flow. So air in the laundry treatment barrel 2 is quickly discharged into the drying air duct 9, and sterilized and deodorized by the photoplasma tube 34.

In the embodiment, a control device 10 is also provided. The control device 10 is connected with the photoplasma tube 34 to control the photoplasma tube 34 to irradiate the hot air in the drying air duct 9.

In the embodiment, as an example that the laundry treatment barrel 2 is a single barrel, laundry to be treated is placed inside the laundry treatment barrel 2.The hot air in the drying air duct 9 is directly blown into the laundry treatment barrel 2 through the air duct outlet 92 of the drying air duct 9 and flows from the front of the laundry treatment barrel 2 to the interior of the laundry treatment barrel2, so as to sterilize and deodorize the interior of the laundry treatment barrel 2 and laundry. Humid air in the laundry treatment barrel2is discharged into the drying air duct 9 through the air duct inlet 91 of the drying air duct 9, so that the circulating air flow is irradiated by the photoplasma tube 34.

In the embodiment, as an example that the laundry treatment barrel 2 includes an outer barrel 21 and an inner barrel 22, two ends of the drying air duct 9 are connected with the two ends of the outer barrel 21.The hot air in the drying air duct 9 is blown into the outer barrel 21 through the air duct outlet 92 of the drying air duct 9. A front of the outer barrel 21 and a front of the inner barrel 22 all have openings. The hot air flows into the inner barrel 22 from top to bottom to sterilize and deodorize the interior of the inner barrel 22 and laundry. The hot air in the inner barrel 22 flows downward and into the outer barrel 21 from the opening of the front of the inner barrel 22.The humid air in the outer barrel 21 is discharged into the drying air duct 9 through the air duct inlet 91 of the drying air duct 9, and the circulating air flow is irradiated by the photoplasma tube 34.

### Embodiment 12

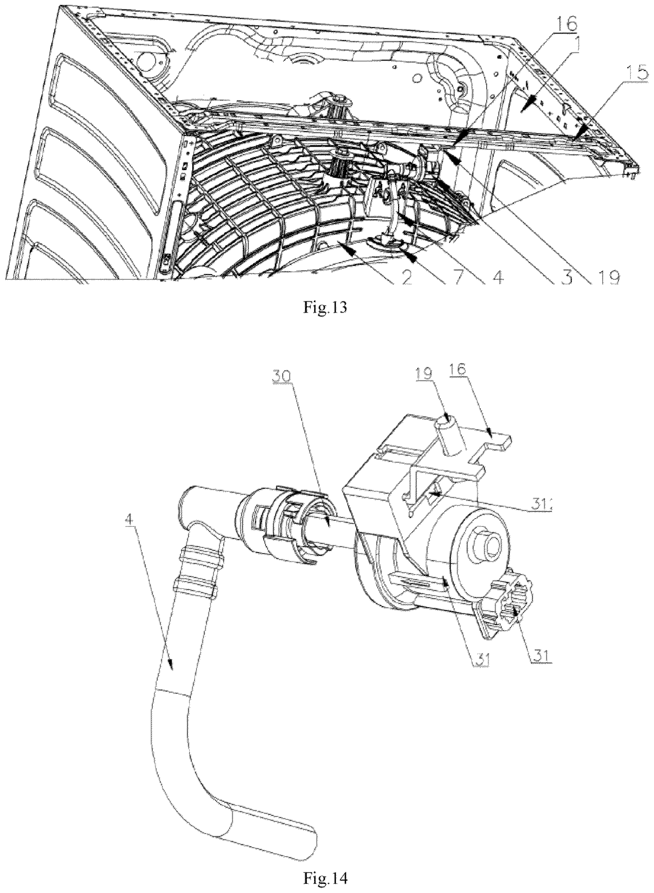

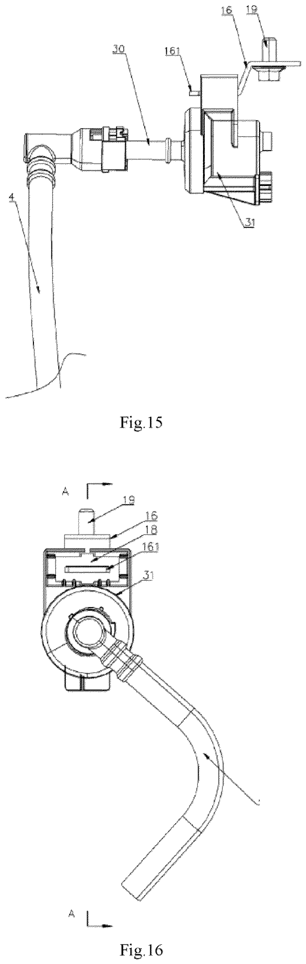

As shown in Figs. 13 to 18, an optical plasma generation unit 3 in the embodiment of the present application, includes: a shell 31 in which an air chamber 32 is formed; a photoplasma tube 34 for irradiating air in the air chamber 32; a connecting pipeline 30; and a blocking member 39. An air inlet end of the connecting pipeline 30 is connected with an air outlet 36 of the air chamber 32. The blocking member 39 is controlled to be connected with or disconnected from the air chamber 32. The blocking member 39 can reciprocate along the inside of the connecting pipeline 30.

In the embodiment, under the action of air flow, the blocking member 39 can reciprocate along the inside of the connecting pipeline 30 to control the connecting pipeline 30 to be connected with or disconnected from the air chamber 32. The structure of the blocking member 39 is simple and easily operated, reducing an amount of parts and production costs.

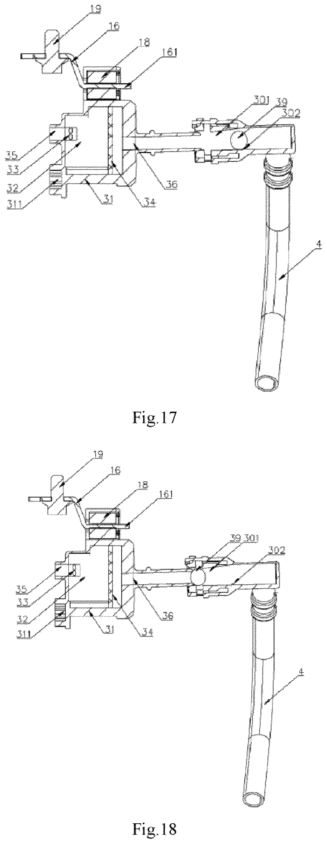

As shown in Fig. 17 and Fig. 18, in the embodiment, the connecting pipeline 30 includes an inclined pipeline section 301 extending obliquely upward along an air flow direction. The blocking member 39 is arranged in the inclined pipeline section 301. The blocking member 39 is configured to move upward under the action of air flow, such that the connecting pipeline 30 is connected with air chamber 32. Or the blocking member 39 is configured to move downward under the action of gravity, such that the connecting pipeline 30 is disconnected from the air chamber 32.

As shown in Figs. 17 and 18, in the embodiment, the air chamber 32 is provided with a fan 33 for guiding air in the air chamber 32 to flow into the connecting pipeline 30. Air flows through the inclined pipeline section 301 to drive the blocking member 39 to move upwards, so that the connecting pipeline 30 is communicated with the air chamber 32. There is no need to use a valve assembly to control the on-off of the connecting pipeline 30, and it is more precise and simple to be controlled.

As shown in Fig. 17 and Fig. 18, in the embodiment, an inner peripheral wall of the inclined pipeline section 301 is provided with a limiting rib 302 extending in a radial direction of the inside of the inclined pipeline section 301. Air flows through the inclined pipeline section 301 to drive the blocking member 39 to move upward and be in contact with the limiting rib 302, so that the blocking member 39 is stopped moving upward.

As an embodiment, the inner peripheral wall of the inclined pipeline section 301 is provided with a circle of the limiting rib 302 extending in a radial direction of the inside of the inclined pipeline section 301. The limiting rib 302 is configured to be contact with blocking member 39. The blocking member 39 moves upwards under the action of the air flow until the blocking member 39 is in contact with the limiting rib 302. The blocking member 39 stops moving upwards, and the sterilizing gas is discharged from the gap between the blocking member 39 and the connecting pipeline 30.

As shown in Figs. 17 and 18, in the embodiment, an inner diameter of the inclined pipeline section 301 is gradually increased in the air flow direction. The sterilizing gas generated by irradiating air in the air chamber 32 by the photoplasma tube 34 can flow out more smoothly of the gap between the blocking member 39 and the connecting pipeline 30.

As an embodiment, the inclined pipeline section 301 is a conical pipeline extending obliquely upwards, such that the sterilizing gas flows easily out of the connecting pipeline 30, thereby sterilizing and deodorizing the interior of the laundry treatment device.

As shown in Fig. 14, part of the shell 31 is recessed toward the interior of the air chamber 32 to form an accommodating chamber 312 having an opening in a horizontal direction. An elastic member 18 is arranged in the accommodating chamber 312 and in interference fit with the accommodating chamber 312. An insertion hole is formed in the elastic member 18. The accommodating chamber 312 is provided with a mounting groove for the elastic member 18. The elastic member 18 is made of materials such as rubber, silicone or other elastic materials.

As shown in Fig. 17 and Fig. 18, in the embodiment, the blocking member 39 can be a spherical shape, and is made of materials such as rubber, silicone, plastic, metal.

### Embodiment 13

As shown in Fig. 13, a laundry treatment apparatus in an embodiment of the present application, includes: a housing 1, a laundry treatment barrel 2 arranged in the housing 1, and an optical plasma generating unit 3 as described above. An air outlet end of the connecting pipeline 30 is communicated with the interior of the laundry treatment barrel 2 through the air outlet pipeline 4.

As shown in Figs. 13 to 15, in the embodiment, the air inlet end of the air outlet pipeline 4 is connected with the air chamber 32 through the connecting pipeline 30. A door seal 5 is arranged between the housing 1 and a front flange of the laundry treatment barrel 2. An air outlet end of the air outlet pipeline 4 is connected with an upper front side of the laundry treatment barrel 2 or the door seal 5, so that the circulating air irradiated by the photoplasma tube 34 in the air chamber 32 is blown into the interior of the laundry treatment barrel 2 through the air inlet pipeline 6.

As shown in Fig. 13, in the embodiment, the air inlet end of the air outlet pipeline 4 is connected with the optical plasma generation unit 3, and the air outlet end of the air outlet pipeline 4 is connected with the upper front side of the laundry treatment barrel 2. The sterilizing gas from by the optical plasma generation unit 3 is blown into an air inlet pipeline 6, and then flows into the laundry treatment barrel 2 from the front thereof.

As shown in Fig. 13, in the embodiment, an air inlet end of the air outlet pipeline 4 is connected with the optical plasma generation unit 3, and an air outlet end of the air outlet pipeline 4 is connected with the door seal 5. The sterilizing gas from the optical plasma generation unit 3 is blown into the air outlet pipeline 4, and then flows into the laundry treatment barrel 2 through the door seal 5.

In the embodiment, a radial dimension of the air inlet pipeline 6 is larger than a radial dimension of the air outlet pipeline 4, which is conducive to the rapid flow of the circulating air flow. So the air flow in the air inlet pipeline 6 can flow quickly into the air chamber 32, and the air flow in the laundry treatment barrel 2 is sterilized and deodorized by the optical plasma generation unit 3.

As shown in Figs. 13 to 15, in the embodiment, the connecting pipeline 30 is fixedly connected with the air outlet pipeline 4 by buckle, clamp, and so on. This connection type is more convenient to be disassembled and assembled.

As shown in Fig. 13, in the embodiment, a beam 15 is arranged on a top of the housing 1. The beam is fixedly connected with a mounting bracket 16. The shell 31 of the optical plasma generation unit 3 is detachably connected with the mounting bracket 16 through a plug-in structure, so the optical plasma generation unit 3 is fixedly connected with the housing 1 via the mounting bracket 16.

As an embodiment, the beam 15 and the mounting bracket 16 are respectively provided with a connecting hole. A fastener 19 passes through the connecting hole on the beam 15 and the connecting hole on the mounting bracket 16 to connect the beam 15 with the mounting bracket 16.

As shown in Figs. 13 to 15, in the embodiment, part of the shell 31 is recessed toward the interior of the air chamber 32 to form an accommodating chamber 312 being in interference fit with the elastic member 18. The elastic member 18 is provided with an insertion hole. The mounting bracket 16 is provided with a stick 161 matching with the insertion hole.

In the embodiment, compared with the optical plasma generation unit 3 directly being connected with the mounting bracket 16, the mounting bracket 16 is fixedly connected with the elastic member 18 having a certain buffering effect through a plug-in structure, which can reduces the impact of vibration of the laundry treatment apparatus on the photoplasma tube 34.

As shown in Fig. 13, Fig. 16 and Fig. 17, in the embodiment, the shell 31 is recessed inwardly to form a terminal card slot 311 for installing a terminal. The terminal is connected with a controller of the laundry treatment apparatus to control the photoplasma tube 34 to irradiate air in the gas chamber 32 .

In the embodiment, the laundry treatment apparatus may be a washing machine, a laundry dryer or other apparatus with a laundry treatment function. A laundry treatment barrel 2 can be a single barrel, and can also include an outer barrel and an inner barrel being communicated with each other.

Through the above-mentioned laundry treatment apparatus, a sterilizing gas is generated by irradiating air in the air chamber 32 by the photoplasma tube 34, and flows into the interior of the laundry treatment barrel 2 through the air outlet pipeline 4. So both the interior of the laundry treatment barrel 2 and laundry in the laundry treatment barrel 2 can be sterilized, especially a sterilizing effect is better for laundry being no-resistant to high temperature.

The above descriptions are only preferred embodiments of the present application, and do not limit the present application in any form. Although the present application has been disclosed as above with preferred embodiments, it is not intended to limit the present application. Anyone familiar with the technology of this patent Without departing from the scope of the technical solution of the present application, personnel can use the technical content of the above prompts to make some changes or modify them into equivalent embodiments with equivalent changes. In essence, any simple modifications, equivalent changes and modifications made to the above embodiments still fall within the scope of the solutions of the present application.

## Claims

1. A laundry treatment apparatus, including, a laundry treatment barrel and an optical plasma generation unit with an air chamber, wherein,
an air inlet of the air chamber is communicated with an external air, and a photoplasma tube is arranged in the air chamber for irradiating air being in the air chamber,
an air outlet of the air chamber is communicated with an interior of the laundry treatment barrel via an air outlet pipeline.

2. The laundry treatment apparatus according to claim 1, including, a housing, wherein,
the laundry treatment barrel is disposed inside the housing, and the optical plasma generation unit is arranged between the housing and the laundry treatment barrel.

3. The laundry treatment apparatus according to claim 2, wherein, an air inlet end of the air outlet pipeline is connected with the air chamber, and an air outlet end of the air outlet pipeline is connected with an upper front side of the laundry treatment barrel, such that the external air in the air chamber irradiated by the photoplasma tube is blown into the laundry treatment barrel through the air outlet pipeline.

4. The laundry treatment apparatus according to claim 3, wherein, a first air inlet is provided on the upper front side of the laundry treatment barrel, and the air outlet pipeline is communicated with an interior of the laundry treatment barrel through the first air inlet;
preferably, the air outlet pipeline is provided with an air outlet valve for controlling to open and close the air outlet pipeline.

5. The laundry treatment apparatus according to claim 2, wherein, a door seal is arranged between the housing and a front flange of the laundry treatment barrel, an air outlet end of the air outlet pipeline is connected with the door seal, such that the external air in the air chamber irradiated by the photoplasma tube is blown into the laundry treatment barrel through the air outlet pipeline.

6. The laundry treatment apparatus according to claim 5, wherein, a front of the laundry treatment barrel has an opening, an upper of the door seal corresponding to an upper front side of the laundry treatment barrel is provided with a second air inlet, and the air outlet pipeline is communicated with the opening on the front of the laundry treatment barrel through the second air inlet;
preferably, the air outlet pipeline is provided with an air outlet valve for controlling to open and close the air outlet pipeline.

7. The laundry treatment apparatus according to any one of claims 1 to 6, wherein, a fan is arranged in the air chamber for taking external air into the air chamber through the air inlet of the air chamber, and discharging the external air in the air chamber irradiated by the photoplasma tube into the air outlet pipeline through the air outlet of the air chamber;
preferably, the air chamber is connected with an air inlet valve for controlling to open and close the air inlet of the air chamber, and/or an air outlet valve for controlling to open and close the air outlet of the air chamber.

8. The laundry treatment apparatus according to claim 7, wherein, the fan is arranged on a position close to the air inlet of the air chamber, and the photoplasma tube is arranged on a position close to the air outlet of the air chamber.

9. A laundry treatment apparatus, including, a housing, a laundry treatment barrel disposed in the housing, and an optical plasma generation unit with an air chamber, wherein,
an air inlet and an air outlet of the air chamber are communicated with an interior of the laundry treatment barrel through an air inlet pipeline and an air outlet pipeline respectively to form a circulating air flow, and
a photoplasma tube for irradiating the circulating air flow is disposed in the air chamber.

10. The laundry treatment apparatus according to claim 9, wherein,
an air inlet end of the air inlet pipeline is connected with a upper rear side of the laundry treatment barrel, and the air inlet end of the air inlet pipeline is connected with the air chamber, so that air inside the laundry treatment barrel is discharged into the air chamber through the air inlet pipeline; and
an air inlet end of the air outlet pipeline is connected with the air chamber, a door seal is provided between the housing and a front flange of the laundry treatment barrel, and an air outlet end of the air outlet pipeline is connected with an upper front side of the laundry treatment barrel or the door seal, so that a circulating air in the air chamber irradiated by the photoplasma tube is blown into the laundry treatment barrel through the air outlet pipeline.

11. The laundry treatment apparatus according to claim 10, wherein, a radial dimension of the air outlet pipeline is greater than a radial dimension of the air inlet pipeline.

12. The laundry treatment apparatus according to claim 11, wherein, an air outlet is provided on an upper rear side of the laundry treatment barrel, and the air inlet pipeline is communicated with an interior of the laundry treatment barrel through the air outlet;
preferably, an air inlet valve for controlling to open and close the air inlet pipeline is arranged in the air inlet pipeline.

13. The laundry treatment apparatus according to claim 11, wherein, a first air inlet is provided on the upper front side of a laundry treatment apparatus, and the air outlet pipeline is communicated with the interior of the laundry treatment apparatus through the first air inlet;
preferably, an air outlet valve for controlling to open and close of the air outlet pipeline is arranged in the air outlet pipeline.

14. The laundry treatment apparatus according to claim 11, wherein, an opening is formed on a front of the laundry treatment apparatus, an upper of the door seal corresponding to the upper front of the laundry treatment apparatus is provided with a second air inlet, and the air outlet pipeline is communicated with the opening on the front of the laundry treatment apparatus through the second air inlet.

15. The laundry treatment apparatus according to any one of claims 9 to 14, wherein, a fan is arranged in the air chamber for taking external air into the air chamber through the air inlet of the air chamber, and discharging a circulating air in the air chamber irradiated by the photoplasma tube into the air outlet pipeline through the air outlet of the air chamber.

16. The laundry treatment apparatus according to claim 15, wherein, the air chamber includes a first chamber and a second chamber which are arranged in front and back direction and communicated with each other;
the fan is arranged in the first chamber, and the photoplasma tube is arranged in the second chamber; and
the first chamber has the air inlet of the air chamber, and the second chamber has the air outlet of the air chamber.

17. The laundry treatment apparatus according to claim 16, wherein, the fan is arranged on a position close to the air inlet of the first chamber, and the photoplasma tube is arranged on a position close to the air outlet of the second chamber.

18. The laundry treatment apparatus according to claim 17, wherein, the air outlet of the air chamber is formed on a bottom of the second chamber, and an upper end of the air inlet pipeline is connected with the bottom of the air chamber.

19. A laundry treatment apparatus, including, a laundry treatment barrel and an optical plasma generation unit with an air chamber, wherein,
an air outlet of the air chamber is communicated with the laundry treatment barrel through an air outlet pipeline, an air inlet of the air chamber is communicated with the laundry treatment barrel through a first air inlet branch or communicated with an external air through a second air inlet branch.

20. The laundry treatment apparatus according to claim 19, wherein, the air chamber has a first air inlet and a second air inlet, the first air inlet of the air chamber is communicated with the laundry treatment barrel through the first air inlet branch, and the second air inlet of the air chamber is communicated with the external air through the second air inlet branch.

21. The laundry treatment apparatus according to claim 20, wherein, the first air inlet branch is provided with a first air inlet valve for controlling to open and close the first air inlet branch, and/or
the second air inlet branch is provided with a second air inlet valve for controlling to open and close the second air inlet branch.

22. The clothes processing equipment according to claim 19, wherein, the first air inlet branch has a first air inlet and an air outlet, the first air inlet branch between the first air inlet and the air outlet is provided with a second air inlet for communicating with the external air, and the first air inlet branch between the second air inlet and the air outlet is configured to form the second air inlet branch.

23. The laundry treatment apparatus according to claim 22, including, an external connection pipeline connected with the second air inlet branch, wherein,
the second air inlet is communicated with the external air through the external connection pipeline.

24. The laundry treatment apparatus according to claim 23, wherein, a second air inlet valve is disposed in the second air inlet or in the external connection pipeline to control the second air inlet branch to be connected with or disconnected from the external air;
preferably, the first air inlet branch between the first air inlet and the second air inlet is provided with a first air inlet valve for controlling to open and close the first air inlet branch.

25. The laundry treatment apparatus according to claim 23, wherein, a side of the first air inlet branch away from the second air inlet branch or the second air inlet is provided with a three-way valve, and
the first air inlet branch between the first air inlet and the second air inlet is communicated with the external air and the second air inlet branch through the three-way valve.

26. The laundry treatment apparatus according to any one of claims 19 to 25, wherein one end of the first air inlet branch is connected with an upper rear side of the laundry treatment barrel, and another end is connected with the air chamber; and
one end of the air outlet pipeline is connected with the air chamber, and another end is connected with an upper front side of the laundry treatment barrel or connected with the door seal on the upper front side of the laundry treatment barrel.

27. The laundry treatment apparatus according to any one of claims 19 to 25, wherein, a photoplasma tube and a fan are arranged in the air chamber, the photoplasma tube is configured to irradiate air in the air chamber, and the fan is configured to take air in the first air inlet branch or the second air inlet branch into the air chamber.

28. A laundry treatment apparatus, including, a laundry treatment barrel and a drying air duct for allowing hot air to flow into the laundry treatment barrel, wherein,
both ends of the drying air duct are respectively connected with a front and a rear of the laundry treatment barrel, and the drying air duct is provided with a photoplasma tube for irradiating the hot air in the drying air duct.

29. The laundry treatment apparatus according to claim 28, wherein, the drying air duct is provided with an installation hole, and the photoplasma tube is arranged in the drying air duct by passing a lower end the photoplasma tube through the installation hole.

30. The laundry treatment apparatus according to claim 29, wherein, a fan is arranged in the drying air duct, the drying air duct has an air duct inlet and an air duct outlet, the fan is arranged on a position of the drying air duct close to the air duct inlet, and the photoplasma tube is arranged on a position of the drying air duct close to the air duct outlet.

31. The laundry treatment apparatus according to claim 30, wherein, a heating pipe is arranged in the drying air duct, and between the fan and the photoplasma tube, so that the hot air in the drying air duct flows toward the photoplasma tube.

32. The laundry treatment apparatus according to claim 31, wherein, the drying air duct is provided with an air inlet valve for controlling to open and close the air duct inlet, and the air inlet valve is arranged between the fan and the air duct inlet, so that air in the laundry treatment barrel flows into the drying air duct through the air duct inlet.

33. The laundry treatment apparatus according to claim 32, wherein, the air inlet of the drying air duct is connected with an upper rear side of the laundry treatment barrel, so that air in the laundry treatment barrel flows backward and into the drying air duct through the air duct inlet.

34. The laundry treatment apparatus according to claim 31, wherein, the drying air duct is provided with an air outlet valve for controlling to open and close the air duct outlet, the air outlet valve is arranged between the photoplasma tube and the air duct outlet, so that the hot air in the drying air duct irradiated by the photoplasma tube is discharged into the laundry treatment barrel through the air duct outlet.

35. The laundry treatment apparatus according to claim 34, including a housing, and a laundry treatment barrel arranged in the housing, wherein,
a door seal is arranged between the housing and a front flange of the laundry treatment barrel, the air outlet of the drying air duct is connected with an upper front side of the laundry treatment barrel or the door seal, so that the hot air in the drying air duct irradiated by the photoplasma tube is blown into the laundry treatment barrel through the air duct outlet.

36. The laundry treatment apparatus according to any one of claims 28 to 35, including a control device, wherein, the control device is connected with the photoplasma tube to control the photoplasma tube to irradiate the hot air in the drying air duct.

37. A laundry treatment apparatus, including, a housing, an optical plasma generation unit, and a laundry treatment barrel arranged in the housing, wherein,
the photoplasma generation unit includes:
a shell, in which an air chamber is formed;
a photoplasma tube for irradiating air in the air chamber;
a connecting pipeline, wherein, an air inlet end of the connecting pipeline is connected with an air outlet of the air chamber, an air outlet end of the connecting pipeline is communicated with an interior of the laundry treatment barrel through an air outlet pipeline; and
a blocking member, being configured to control to be connected with or disconnected from the air chamber, such that the blocking member reciprocates along an inside of the connecting pipeline.

38. The laundry treatment apparatus according to claim 37, wherein, the connecting pipeline includes an inclined pipeline section extending obliquely upward along an air flow direction, and the blocking member is arranged in the inclined pipeline section; and
a fan is arranged in the air chamber for guiding air in the air chamber to flow into the connecting pipeline, and air is configured to flow through the inclined pipeline section to drive the blocking member to move upwards, so that the connecting pipeline is communicated with the air chamber.

39. The laundry treatment apparatus according to claim 38, wherein, an inner peripheral wall of the inclined pipeline section is provided with a limiting rib extending in a radial direction of the inside of the inclined pipeline section, and air is configured to flow through the inclined pipeline section to drive the blocking member to move upward and be in contact with the limiting rib, so that the blocking member is stopped moving upward.

40. The laundry treatment apparatus according to claim 38, wherein, an inner diameter of the inclined pipeline section is gradually increased in an air flow direction.

41. The laundry treatment apparatus according to any one of claims 37 to 40, wherein, part of the shell is recessed toward an interior of the air chamber to form an accommodating chamber having an opening in a horizontal direction, an elastic member is arranged in the accommodating chamber and in interference fit with the accommodating chamber, and an insertion hole is formed in the elastic member.

42. The laundry treatment apparatus according to any one of claims 37 to 40, wherein, a photocatalyst excitation layer is in the shell, and the photoplasma tube is in opposite to the photocatalyst excitation layer; or
a photocatalyst excitation layer is provided inside the photoplasma tube.

43. The laundry treatment apparatus according to claim 42, wherein, an ultraviolet tube has UVC ultraviolet bands and/or UVD ultraviolet bands.

44. The laundry treatment apparatus according to claim 37, wherein, a beam is arranged on a top of the housing, and fixedly connected with a mounting bracket,
the shell of an optical plasma generation unit is detachably connected with the mounting bracket through a plug-in structure;
preferably, the beam and the mounting bracket are respectively provided with a connecting hole, a fastener is configured to pass through the connecting hole on the beam and the connecting hole on the mounting bracket to connect the beam with the mounting bracket.

45. The laundry treatment apparatus according to claim 44, wherein, part of the shell is recessed toward an interior of the air chamber to form an accommodating chamber being in interference fit with the elastic member, the elastic member is provided with an insertion hole, and the mounting bracket is provided with a stick matching with the insertion hole.
